# EUROPÄISCHE PATENTSCHRIFT

(11) **EP 3 612 245 B1**
(45) Veröffentlichungstag und Bekanntmachung des Hinweises auf die Patenterteilung: **17.12.2025**
(21) Anmeldenummer: 18719831.2
(22) Anmeldetag: 20.04.2018
(51) Int. Cl.: A61M 1/16

(54) **STOFFAUSTAUSCHVORRICHTUNG**
SUBSTANCE EXCHANGE DEVICE
DISPOSITIF D'ÉCHANGE DE SUBSTANCES

(30) Priorität: 20.04.2017 AT 503182017
(43) Veröffentlichungstag der Anmeldung: 26.02.2020
(73) Patentinhaber: Technische Universität Wien, 1040 Wien (AT)
(72) Erfinder: GFÖHLER, Margit, 1230 Wien (AT); JANECZEK, Christoph, 2603 Felixdorf (AT); HARASEK, Michael, 1160 Wien (AT)
(74) Vertreter: SONN Patentanwälte GmbH & Co KG
(86) Internationale Anmeldenummer: PCT/EP2018/060160
(87) Internationale Veröffentlichungsnummer: WO 2018/193079

(56) Entgegenhaltungen:
- WO-A2-2004/016300
- DE-A1- 102006 036 948
- US-A1- 2013 053 623

## Beschreibung

Die Erfindung betrifft eine Stoffaustauschvorrichtung zur intravenösen Verwendung umfassend einen Hohlraum zur Aufnahme von Blut mit zumindest einem Bluteinlass und zumindest einem Blutauslass, eine an den Hohlraum angrenzende Stoffaustauschmembran, eine Zuführleitung zur Zuführung eines Austauschfluids zu der Stoffaustauschmembran, eine in dem Hohlraum angeordnete Blutpumpe und eine Antriebseinheit für die Blutpumpe, wobei die Blutpumpe zum Pumpen von Blut in Richtung vom Bluteinlass zum Blutauslass des Hohlraums eingerichtet ist.

Als Stoffaustauschvorrichtung ist in diesem Zusammenhang jede Vorrichtung zum Austausch von Substanzen aus dem Blut oder in das Blut zu verstehen. Die Stoffaustauschmembran kann eine erste Seite und eine der ersten Seite gegenüber liegende zweite Seite aufweisen, wobei die erste Seite an den Hohlraum angrenzen kann und wobei die Zuführleitung zur Zuführung des Austauschfluids zu der zweiten Seite der Stoffaustauschmembran eingerichtet sein kann. Eine im Austauschfluid enthaltene oder diesem entsprechende Austauschsubstanz kann über die Membran in das Blut auf der anderen Seite übertreten oder umgekehrt kann eine Austauschsubstanz aus dem Blut in das Austauschfluid übertreten. Mit der Blutpumpe kann im Betrieb eine lokale Druckdifferenz zwischen dem Bluteinlass und dem Blutauslass aufgebaut werden und so den benötigten Blutfluss über die Membran ermöglichen. Aufgrund des Strömungswiderstandes durch die Membran würde sich sonst kaum ein Blutfluss über diese einstellen. Dabei wird der durch die Stoffaustauschvorrichtung verursachte Druckverlust durch die Blutpumpe zumindest teilweise kompensiert. Insbesondere kann mit der Blutpumpe der Blutfluss über die Membran ermöglicht und entlang der Stoffaustauschvorrichtung verbessert, und optional über einen stromaufwärts der Membran angeordneten Bypass unterstützt werden.

Derartige Stoffaustauschvorrichtung sind in unterschiedlichen Ausführungsvarianten aus der Patentliteratur bekannt. So offenbart beispielsweise die WO 2004/016300 A2 einen als Katheter ausgeführten intravenösen Oxygenator zur Sauerstoffanreicherung von Blut mit einer Membran in Form eines Faserbündels, wobei die Fasern jeweils mit einem ersten Anschluss an eine Gaszufuhr und mit einem zweiten Anschluss an einen Gasabzug angeschlossen sind. Das Faserbündel ist tordiert durch eine relative Verdrehung des ersten Anschlusses der Fasern gegenüber dem zweiten Anschluss der Fasern im Betrieb um die Längsachse des Oxygenators. Die Fasern verlaufen daher über die gesamte Länge des Faserbündels als durchgehende Gasleitungen. Im Betrieb des Oxygenators wird Sauerstoff zugeführt, welcher über den ersten Anschluss in die Fasern strömt, an deren Oberfläche ein diffuser Gasaustausch mit Blut stattfindet. Dabei kommt es zu einer Anreicherung des Bluts mit Sauerstoff bei gleichzeitiger Entfernung von CO₂. Am zweiten Anschluss befindet sich in den Fasern daher ein Gasgemisch aus Sauerstoff und Kohlendioxid, welches durch eine Abzugkammer in einem Schlauch und durch diesen aus dem Körper des Patienten herausströmt. Das in den Oxygenator strömende Blut durchströmt das tordierte Faserbündel und gelangt zu einer Pumpe. Das Blut wird dort in Fließrichtung der Vene befördert und verlässt den Oxygenator durch einen Auslass. Der Druckabfall des Blutes wird daher durch die Pumpe ausgeglichen, sodass der Druck am Auslass wieder den physiologischen Druck aufweist.

Die US 2010/258116 A1 erwähnt nur am Rande die Verwendung eines Impellers, allerdings ohne Hinweis auf eine Verwendung als Blutpumpe. Der Impeller ist nicht näher dargestellt und es wird keine ausführbare technische Lösung dazu angegeben, wie ein solcher Impeller angeordnet und angetrieben werden könnte, sodass die Funktion des Impellers unklar bleibt.

Die in US 2013/053623 A1 gezeigte Pumpe dient der Unterstützung der Herzfunktion und betrifft somit ein gänzlich anderes Anwendungsgebiet als die vorliegende Vorrichtung. Die gezeigte Pumpe umfasst naturgemäß keine Membran im Sinn der erfindungsgemäßen Vorrichtung, d.h. zum Austausch von Substanzen.

Bei der in GB 2505068 A gezeigten Vorrichtung wird eine Blutpumpe über eine Antriebswelle von einer außerhalb des Körpers angeordneten Antriebseinheit angetrieben. D.h. die Antriebseinheit ist in diesem Fall nicht Teil der Stoffaustauschvorrichtung zur intrakorporalen Verwendung.

Die DE 10 2006 036 948 A1 zeigt eine Blutpumpe und eine extrakorporale Verwendung der Blutpumpe, wobei diese von einer über eine Magnetkupplung verbundenen Turbine angetrieben wird und Blut durch einen ebenfalls extrakorporalen Oxygenator pumpt.

Es ist eine Aufgabe der Erfindung, eine separate Energieversorgung der Antriebseinheit zu vermeiden.

Diese Aufgabe wird durch eine Stoffaustauschvorrichtung der eingangs angeführten Art erfindungsgemäß dadurch erzielt, dass die Antriebseinheit eine Turbine umfasst, welche mit der Zuführleitung verbunden und mittels eines durch die Zuführleitung zugeführten Austauschfluids antreibbar ist, wobei die Turbine mindestens ein mit der Blutpumpe gekoppeltes Laufrad und ein vor dem Laufrad angeordnetes Leitrad aufweist. D.h. das Leitrad ist bezüglich der Strömungsrichtung des Austauschfluids im Betrieb stromaufwärts des Laufrads angeordnet. Mit dem vor dem Laufrad angeordneten Leitrad wird eine richtige Anströmung des Laufrades der Turbine von dem das Leitrad durchströmende Austauschfluids (z.B. einer Austauschsubstanz oder einem Trägermedium) erzielt. Ein Teil der inneren Energie des strömenden Austauschfluids wird mit der Turbine in mechanische Leistung umgewandelt, welche über eine Welle an die Blutpumpe abgegeben wird. Das Austauschfluid kann flüssig oder gasförmig sein. Es kann durch einen Katheter von außerhalb des Körpers der Stoffaustauschvorrichtung zugeführt werden, und durch eine extrakorporale Pumpe durch die Stoffaustauschvorrichtung gefördert werden. Das Austauschfluid kann eine flüssige oder gasförmige Austauschsubstanz oder ein flüssiges oder gasförmiges Trägermedium oder Fördermedium sein, in dem die Austauschsubstanz gelöst ist bzw. mit dem die Austauschsubstanz gemischt ist bzw. von dem die Austauschsubstanz aus dem Blut aufgenommen wird.

Eine Aufgabe der Antriebseinheit ist die Umwandlung von über das Fluid zugeführter Energie in ein auf eine Welle übertragenes Drehmoment innerhalb der Stoffaustauschvorrichtung. Die Energieversorgung der Antriebseinheit erfolgt somit zusammen mit der Zuführung des Austauschfluids und nicht separat bzw. unabhängig davon. D.h. sowohl die Blutpumpe als auch die Turbine sind in der Stoffaustauschvorrichtung integriert. Die Achse der Welle ist im Wesentlichen parallel zu einer Längserstreckungsachse der Stoffaustauschvorrichtung angeordnet. Die Welle überträgt das in der Antriebseinheit erzeugte Drehmoment auf einen mit der Antriebseinheit verbundenen Pumpenläufer der Blutpumpe. Dieser überträgt das Drehmoment schließlich auf das Blut. Bei einer solchen Fördereinrichtung umfassend eine Turbine als Antriebseinheit, und eine Blutpumpe als Arbeitsmaschine wird ein Teil der Strömungsenergie des Austauschfluids auf das geförderte Blut übertragen.

Zusätzlich zur Zuführleitung kann die Stoffaustauschvorrichtung eine Rückführleitung zur Rückführung des Austauschfluids von der Stoffaustauschmembran aufweisen. Die vorliegende Erfindung ist nicht auf jeweils eine Zuführleitung und eine Rückführleitung beschränkt. Insbesondere können zwei Zuführleitungen und/oder zwei Rückführleitungen vorgesehen sein, wobei beispielsweise unterschiedliche Zuführleitungen und/oder unterschiedliche Rückführleitungen mit der Membran respektive mit der Turbine verbunden sein können. Damit kann eine unabhängige Ansteuerung von Stoffaustauschmembran und Turbine erzielt werden. Insbesondere können in diesem Fall die Systeme Turbine und Stoffaustauschmembran auch dann unabhängig voneinander geregelt werden, wenn etwaige angesteuerte Ventile sich nicht im Körper befinden, sondern in einer externen Steuerungseinheit angeordnet sind.

Die Stoffaustauschvorrichtung kann in Verbindung mit einer extrakorporalen Förder- und Austauscheinrichtung zur Wiederaufbereitung und Förderung des Austauschfluids als Gesamtsystem unter Verwendung von bereits auf dem Markt befindlichen Komponenten so dimensioniert werden, dass es tragbar ist und der extrakorporale Kreislauf mit oder an der Konsole sogar in eine Tasche passt.

Im einfachsten und zuverlässigsten Fall ist das Turbinenelement direkt mit dem Pumpenläufer gekoppelt, sodass die beiden Elemente im Betrieb mit derselben Drehzahl laufen. Auch im Fall einer indirekten Kopplung, z.B. mittels einer Magnetkupplung (siehe unten), können die Blutpumpe und die Turbine mit derselben Drehzahl betrieben werden, z.B. bei Verwendung der selben Polpaarzahl der Magneten in der Kupplung. Durch Auslegung der Blutpumpe und der Turbine auf einen gewünschten Betriebspunkt (Drehzahl, Druckverhältnisse, Volumenströme), kann in beiden Fällen ein optimierter Betrieb der Stoffaustauschvorrichtung erreicht werden.

Es ist günstig, wenn ein Pumpenläufer der Blutpumpe in einem Gleitlager gelagert ist. Dabei wird das Blut selbst als Schmiermittel verwendet. Dieser Vorteil kann unabhängig von der Anordnung von Laufrad und Leitrad der Turbine erzielt werden.

Weiteres kann ein Pumpenläufer weitere Teile der Blutpumpe aus einer technischen Keramik wie Aluminiumoxid, aus einem Metall wie Titan oder aus einem Kunststoff wie PEEK (Polyetheretherketon) hergestellt und vorzugsweise zusätzlich mit einer Oberflächenbeschichtung ausgestattet sein. Diese Materialien eignen sich besonders für die Verwendung im Blutkreislauf.

Außerdem ist es vorteilhaft, wenn in der Stoffaustauschvorrichtung zumindest ein Drehzahlsensor integriert ist, welcher zur Aufnahme der Drehzahl der Turbine, der Blutpumpe oder einer Kupplung zwischen Blutpumpe und Turbine eingerichtet ist. Integriert bedeutet in diesem Zusammenhang, dass der Drehzahlsensor Teil der Stoffaustauschvorrichtung ist und demzufolge im Betrieb der Stoffaustauschvorrichtung intrakorporal angeordnet ist. Es können mehrere Drehzahlsensoren vorgesehen sein, sodass die Drehzahl an mehreren der genannten Stellen aufgenommen werden kann. Die aufgenommene Drehzahl kann anschließend in ein elektronisches Signal umgewandelt und zur Überwachung und Kontrolle des ordnungsgemäßen Betriebs der Stoffaustauschvorrichtung verwendet werden. Als Drehzahlsensor kann insbesondere ein Hallsensor vorgesehen sein.

In einer Ausführungsform der vorliegenden Erfindung kann die Blutpumpe über ein Getriebe mit der Turbine gekoppelt sein, wobei das Getriebe zur Herabsetzung der Drehzahl der Blutpumpe gegenüber der Drehzahl der Turbine eingerichtet ist. Eine solche Herabsetzung ist insbesondere bei der Verwendung eines gasförmigen Austauschfluids vorteilhaft, damit die Turbine mit einer entsprechend höheren Drehzahl als für die Blutpumpe erforderlich betrieben werden kann. Generell kann mithilfe des Getriebes ein für das jeweilige Austauschfluid geeigneter Betriebspunkt der Turbine bei vorgegebener Drehzahl der Blutpumpe erreicht werden.

Weiters hat es sich als günstig herausgestellt, wenn die gegenständliche Stoffaustauschvorrichtung eine oder mehrere Rückführleitung(en) zur Rückführung eines Austauschfluids von der Stoffaustauschmembran und/oder der Turbine aufweist, wobei die Rückführleitung eingerichtet ist, einem Unterdruck (d.h. einem gegenüber einem Umgebungsdruck von ca. 1 bar negativem Differenzdruck) stand zu halten. In diesem Fall kann der Überdruck der Zuführleitung reduziert oder gänzlich vermieden werden, wodurch die Gefahr eines Austritts von Austauschfluid in das Blut im Fall eines Lecks verringert werden kann. Es kann auch ein Konzentrationsgefälle an der Stoffaustauschmembran erreicht werden indem anstelle des Austauschfluids ein Vakuum aufgebracht wird.

Im Zusammenhang mit der Antriebseinheit ist es günstig, wenn der Pumpenläufer über eine Magnetkupplung mit der Antriebseinheit verbunden ist, wobei die Magnetkupplung zur Drehmomentübertragung entlang einer Drehachse zwei relativ zueinander drehbare Kupplungsteile mit jeweils einem Dauermagnet aufweist. Als Magnetkupplung kann beispielsweise eine Zentraldrehkupplung oder eine Stirndrehkupplung verwendet werden. Eine Magnetkupplung hat gegenüber einer durchgehenden mechanischen Verbindung, z.B. in Form einer durchgehenden Welle, den Vorteil, dass das übertragene Drehmoment begrenzt ist und eine hermetische Trennung zwischen blutführenden und nicht blutführenden Teilen hergestellt werden kann. Wenn die Turbine stecken bleibt, bleibt zwar auch der Pumpenläufer stehen; die Begrenzung ist aber dennoch technisch sinnvoll, da ein Ausfall detektiert werden kann. Sollte die Turbine aufgrund eines Fehlers hochdrehen, würde die Magnetkupplung irgendwann nicht mehr die Leistung übertragen. Der Blutkreislauf ist so vor einer Überlast geschützt.

Um auch bei einer besonders kompakten Magnetkupplung ein gewünschtes Drehmoment übertragen zu können, hat es sich als vorteilhaft herausgestellt, wenn einer der Kupplungsteile ein zumindest teilweise ferromagnetisches Umleitelement umfasst, welches mit dem Dauermagnet des Kupplungsteils drehfest verbunden ist, wobei ein Teil des Umleitelements radial außerhalb des Dauermagnets des anderen Kupplungsteils angeordnet ist. Eine derartige Magnetkupplung ist beispielsweise in der WO 2015/172173 A2 gezeigt, deren Inhalt hiermit in diese Anmeldung aufgenommen wird. D.h. die Magnetkupplung umfasst zwei relativ zueinander drehbare Kupplungsteile, wobei ein antriebsseitiger Kupplungsteil einen antriebsseitigen Dauermagnet aufweist und ein abtriebsseitiger Kupplungsteil einen dem antriebsseitigen Dauermagnet entlang der Drehachse gegenüberliegenden und im Abstand davon angeordneten abtriebsseitigen Dauermagnet aufweist, wobei einer der Kupplungsteile ein zumindest teilweise ferromagnetisches Umleitelement umfasst, welches mit dem Dauermagnet des Kupplungsteils drehfest verbunden ist, wobei ein Teil des Umleitelements radial außerhalb des gegenüberliegenden Dauermagnets angeordnet ist. Diese Bauform hat gegenüber herkömmlichen Zentraldrehkupplungen den Vorteil, dass sie einfacher und kostengünstiger herstellbar ist und eine insgesamt geringere Kupplungsfläche erfordert, da ein Teil des Drehmoments über die Stirnseite der Kupplungsteile übertragen wird. Gegenüber herkömmlichen Stirndrehkupplungen hat sie den Vorteil, dass zur Übertragung eines bestimmten Drehmoments geringere radiale Abmessungen erforderlich sind. Das Umleitelement kann dabei - vergleichbar dem äußeren Kupplungsteil einer Zentraldrehkupplung - topf- bzw. hohlzylinderförmig ausgebildet sein und den jeweils anderen Kupplungsteil umfangseitig umgeben, d.h. es erstreckt sich vorzugsweise radial außerhalb beider Dauermagnete. Dabei kann das Umleitelement beispielsweise als dünnwandiger Hohlzylinder ausgebildet sein, sodass bei gleichbleibenden Abmessungen das magnetisierte Volumen der Stirndrehkupplung weitestgehend erhalten bleibt und zugleich zwischen dem Umleitelement und dem im Abstand davon gegenüberliegenden Dauermagnet ein einer Zentraldrehkupplung vergleichbar großes übertragbares Drehmoment erzielbar ist. Die Magnetisierungsrichtung der Dauermagnete ist dabei vorzugsweise senkrecht auf die Drehachse ausgerichtet, d.h. die Magnetpole verlaufen in Umfangsrichtung von Süd nach Nord und liegen sich - jedenfalls bei einer zweipoligen Ausführung - bezüglich der Drehachse diametral gegenüber. Durch das Umleitelement werden radial von den Dauermagneten ausgehende magnetische Feldlinien gebündelt und die magnetische Kraft zwischen den Kupplungsteilen wegen des ferromagnetischen Materials des Umleitelements zusätzlich verstärkt. Durch die Verdichtung der magnetischen Feldlinien im ferromagnetischen Material wird die magnetische Kraft zur Übertragung des Drehmoments vergrößert. Aufgrund des im Vergleich zu Zentraldrehkupplungen bei gleichen Kupplungsabmessungen größeren Volumens der Dauermagneten kann vorteilhafter Weise eine geringere axiale Erstreckung und somit geringere radiale Querkräfte auf die Lager der Kupplungsteile erzielt werden.

Die Dauermagnete der Magnetkupplung können jeweils 2-, 4-, oder 6- polige Dauermagnete sein. Sie sind vorzugsweise zweipolig mit jeweils zwei halbzylinderförmigen Magnetpolen. Das zumindest teilweise ferromagnetische Umleitelement kann zumindest eine diamagnetische oder paramagnetische Trennung aufweisen. Diese Trennung unterteilt das Umleitelement in zumindest zwei ferromagnetische Abschnitte und vermeidet so einen magnetischen Kurzschluss. Es kann eine diamagnetische Trennung oder eine paramagnetische Trennung (z.B. aus Aluminium oder Messing) verwendet werden. Als paramagnetisch werden Materialien mit einer magnetischen Permeabilität von nur wenig größer als 1 bezeichnet, insbesondere mit einer magnetischen Permeabilität kleiner als 1,2, vorzugsweise mit einer magnetischen Permeabilität kleiner als 1,05.

Zusätzlich zur radial äußeren Anordnung kann sich das Umleitelement auch an eine dem gegenüberliegenden Dauermagnet abgewandten Rückseite des drehfest verbundenen Dauermagnets erstrecken. Alternativ oder zusätzlich kann das Umleitelement einen im Wesentlichen H-förmigen Längsschnitt aufweisen, mit einem senkrecht auf die Drehachse liegenden Quersteg und beidseitig topfförmigen Ausnehmungen, wobei in einer dieser Ausnehmungen ein Dauermagnet aufgenommen und drehfest verbunden ist. D.h. das Umleitelement kann einen hohlzylinderförmigen Mantel aufweisen und vorzugsweise mit einem auf im Wesentlichen halber Höhe des Mantels angeordneten Zwischenboden ausgebildet sein.

Eine besonders hohe Konzentration von Magnetfeldlinien im Umleitelement der Magnetkupplung kann erzielt werden, wenn an einer dem gegenüberliegenden Dauermagnet abgewandten Rückseite des drehfest mit dem Umleitelement verbundenen Dauermagnets ein diamagnetisches oder paramagnetisches Abschirmelement angeordnet ist. Dadurch können außerhalb der Kupplungsteile, insbesondere außerhalb des Umleitelementes, verlaufende Feldlinien vermieden und damit verbundene Verluste reduziert werden. D.h. die vom Abschirmelement erzielte "Abschirmung" besteht vorzugsweise darin, dass die magnetischen Feldlinien bevorzugt durch das Umleitelement gehen, als durch das Abschirmelement.

Weiters hat es sich als günstig herausgestellt, wenn bei der Magnetkupplung an einer dem gegenüberliegenden Dauermagnet zugewandten Vorderseite des drehfest mit dem Umleitelement verbundenen Dauermagnets, insbesondere in einem um die Drehachse zentrierten Bereich, ein diamagnetisches oder paramagnetisches Abschirmelement angeordnet ist, welches vorzugsweise umfangseitig bzw. radial außenseitig an das Umleitelement anschließt. Mit einer derartigen Abschirmung kann eine Umlenkung des magnetischen Feldes in radial in größerem Abstand von der Drehachse befindliche Bereiche erzielt werden, sodass das bei gegebener Magnetkraft übertragene Drehmoment erhöht wird.

Um einen Übertritt des Trägermediums in das Blut zuverlässig zu vermeiden, ist es zudem günstig, wenn die beiden Kupplungsteile hermetisch getrennt sind. Eine solche hermetische Trennung kann etwa durch eine zwischen den beiden Kupplungsteilen angeordnete hermetische Trennwand erzielt werden, welche die Antriebseinheit und die Blutpumpe hermetisch voneinander trennt. Die hermetische Trennwand sollte weder magnetisch noch elektrisch leitend sein. Insbesondere kann zumindest einer der Kupplungsteile in einem im Wesentlichen nichtmagnetischen und elektrisch nicht leitenden Gehäuse untergebracht sein, sodass Verluste aufgrund einer Ummagnetisierung des Gehäuses bzw. induzierte Wirbelströme im Gehäuse vermieden werden können.

Vorzugsweise kann das Umleitelement mit dem antriebsseitigen Kupplungsteil drehfest verbunden sein, wobei innerhalb einer hermetischen Trennwand zwischen den beiden Kupplungsteilen zumindest ein Spülkanal vorgesehen ist, welcher einen Spalt zwischen der Stirnseite des pumpenseitigen Kupplungsteils und der hermetischen Trennwand im Betrieb mit mindestens einem Blutstrom außerhalb der hermetischen Trennwand verbindet, wobei dieser Blutstrom entweder ein Blutstrom durch den Bluteinlass oder ein Blutstrom vor dem Bluteinlass sein kann. Der Spülkanal verbindet somit einen blut- und pumpenseitigen Teil der Kupplung und einen Blutstrom außerhalb der Kupplung miteinander. Der Zulauf zum blut- und pumpenseitigen Teil der Kupplung erfolgt über ein kupplungsnahes Gleitlager vor dem blutseitigen Magneten oder über eine Bohrung in der Welle der Pumpe. Es wird also zwischen der Stirnseite des pumpenseitigen Kupplungsteils und durch die hermetische Trennwand hindurch eine Verbindung zu einem Blutstrom außerhalb der Kupplung hergestellt, wobei die Funktion der hermetischen Trennung zwischen blutführenden und nicht-blutführenden Teilen bestehen bleibt. Mittels solcher Spülkanäle können Toträume auf einer Abtriebseite ("Blutseite") innerhalb der Magnetkupplung verringert oder gänzlich vermieden werden.

Es hat sich insbesondere gezeigt, dass eine integrierte Lagerung, vorzugsweise ein Wälzlager zwischen einem antriebsseitigen Kupplungsteil und der hermetischen Trennwand und eine Gleitlagerung zwischen der hermetischen Trennwand und dem abtriebsseitigen Kupplungsteil vorgesehen sind. D.h. der antriebsseitige Kupplungsteil ist gegenüber der hermetischen Trennwand in einem Wälzlager drehbar gelagert und der abtriebsseitige Kupplungsteil ist gegenüber der hermetischen Trennwand in einem Gleitlager drehbar gelagert. Eine derartige Lagerung sorgt für einen stabileren Betrieb und eine erhöhte Laufruhe im Bereich der Kupplung im Vergleich zu stirnseitig ungelagerten Kupplungsteilen, da hier unter anderem die Gefahr besteht, dass der magnetische Topf zu schwingen beginnt und die Drehmomentübertragung abreißt.

Der Stofftransport über die Membran wird bestimmt durch drei Transportwiderstände, den blutseitigen Stoffübergangswiderstand - auch als Konzentrationspolarisation bezeichnet, den Stofftransportwiderstand durch die Membran und den Stofftransportwiderstand in die Aufnehmerphase (Trägersubstanz) auf der Permeatseite der Membran. Wegen der im Wesentlichen laminaren Strömungsverhältnisse wächst die Grenzschichtdicke und damit der Stoffübergangswiderstand mit der Überströmungslänge der Membran. Aufgrund der Stoffeigenschaften des Blutes - der Rheologie, der niedrigen Diffusionskoeffizienten und der Pufferwirkung - gilt es primär, den blutseitigen Stoffübergangswiderstand zu reduzieren. Dies gelingt durch: a) Erhöhung der Überströmgeschwindigkeit und damit Verringerung der Grenzschichtdicke, b) Verbesserung der Strömungsverteilung und Vergleichmäßigung der Verweilzeit, c) geometrische Massnahmen durch kontrollierte Strömungsführung, durch den Einbau von statischen Turbulenzpromotoren, durch die geometrisch bedingte Induzierung sekundärer Strömungsphänomene und durch gezielte Unterbrechung des Grenzschichtaufbaus, d) kontrolliertes Verteilen, Zusammenführen, Mischen und Wiederverteilen des Blutes auf die Membran. Die gegenständliche Erfindung löst dieses Problem durch die Kombination der Maßnahmen a) bis d).

Deshalb ist es besonders vorteilhaft, wenn zwischen der Blutpumpe und dem zumindest einen Blutauslass im Hohlraum eine Umlenkeinrichtung angeordnet ist wobei die Umlenkeinrichtung zur teilweisen Umlenkung eines axial durch den Hohlraum strömenden Blutstroms in radiale Richtung eingerichtet ist und / oder zur Induzierung von Turbulenz in eben diesem Blutstrom eingerichtet ist. Beispielsweise kann die Umleiteinrichtung als statischer Turbulenzpromotor dienen. Durch die teilweise Umlenkung des Bluts in Richtung einer radialen Strömungskomponente und/oder Induzierung von Turbulenz in der Strömung kann der Austausch mit dem Austauschfluid an der Stoffaustauschmembran verbessert werden.

Beispielsweise kann die Umlenkeinrichtung konzentrisch zu einer Längsachse zwischen der Blutpumpe und dem Blutauslass spiralförmige, kegelförmige (bzw. kegelstumpfförmige), pfeilförmige und/oder scheibenförmige Leitflächen aufweisen.

Die Umlenkeinrichtung kann im Hohlraum drehbar gelagert sein.

Sie kann frei drehbar oder vorzugsweise mit dem Pumpenläufer der Blutpumpe für eine erzwungene Drehung der Umlenkeinrichtung gekoppelt sein.

Vorzugsweise kann der Hohlraum zumindest zwei Blutauslässe in unterschiedlichen Abständen von dem zumindest einen Bluteinlass aufweisen. Insbesondere kann ein erster Blutauslass unmittelbar stromabwärts der Blutpumpe und ein zweiter Blutauslass stromabwärts der Stoffaustauschmembran vorgesehen sein. Der erste Blutauslass bildet einen Bypass für die Stoffaustauschmembran. Im Betrieb kann durch den ersten Blutauslass Blut aus dem Hohlraum und aus der Stoffaustauschvorrichtung nach außen in die Umgebung der Stoffaustauschvorrichtung, z.B. in ein umgebendes Gefäß, strömen. Dieses Blut hat aufgrund des Antriebs durch die Blutpumpe eine höhere innere Energie als das an der Stoffaustauschvorrichtung vorbei strömende Blut, sodass die Abgabe zu einem vergleichsweise höheren lokalen Druck im Gefäß führt. Eine derartige Anordnung mehrerer Blutauslässe kann auch unabhängig von der Verwendung einer Turbine als Antriebseinheit vorgesehen sein und demzufolge auch unabhängig von der Anordnung von Laufrad und Leitrad einer Turbine. Anstelle einer Turbine könnte die Stoffaustauschvorrichtung beispielsweise einen Elektromotor als Antriebseinheit für die Blutpumpe umfassen.

Die Erfindung betrifft auch allgemein eine Stoffaustauschvorrichtung zur intravenösen Verwendung umfassend einen Hohlraum zur Aufnahme von Blut mit zumindest einem Bluteinlass und zumindest einem Blutauslass, eine an den Hohlraum angrenzende Stoffaustauschmembran, eine Zuführleitung zur Zuführung eines Austauschfluids zu der Stoffaustauschmembran, eine in dem Hohlraum angeordnete Blutpumpe und eine Antriebseinheit für die Blutpumpe, wobei die Blutpumpe zum Pumpen von Blut in Richtung von einem Bluteinlass zu einem Blutauslass des Hohlraums eingerichtet ist, wobei die Antriebseinheit eine Turbine umfasst, welche mit der Zuführleitung verbunden und mittels eines durch die Zuführleitung zugeführten Austauschfluids antreibbar ist.

Die Erfindung wird nachfolgend anhand von besonders bevorzugten Ausführungsbeispielen, auf die sie jedoch nicht beschränkt sein soll, und unter Bezugnahme auf die Zeichnungen noch weiter erläutert. Dabei zeigen im Einzelnen:
Fig. 1 schematisch einen Längsschnitt durch eine erfindungsgemäße Stoffaustauschvorrichtung bei einer intrakorporalen und intravaskulären Verwendung;
Fig. 2 schematisch eine Stoffaustauschvorrichtung mit einer Umlenkeinrichtung mit spiralförmigen Leitflächen;
Fig. 3 schematisch eine Stoffaustauschvorrichtung mit einer Umlenkeinrichtung mit kegelstumpfförmigen Leitflächen;
Fig. 4 schematisch einen Teil-Längsschnitt durch eine Stoffaustauschvorrichtung mit einer Umlenkeinrichtung gemäß Fig. 3;
Fig. 5 schematisch einen Längsschnitt einer Umlenkeinrichtung mit pfeilförmigen Leitflächen zur Verwendung in einer erfindungsgemäßen Stoffaustauschvorrichtung;
Fig. 6 schematisch einen Längsschnitt einer Umlenkeinrichtung mit scheibenförmigen Leitflächen zur Verwendung in einer erfindungsgemäßen Stoffaustauschvorrichtung;
Fig. 7 schematisch eine Detailansicht der Magnetkupplung der Stoffaustauschvorrichtung gemäß Fig. 1; und
Fig. 8 schematisch eine intrakorporale und extravaskuläre Verwendung einer erfindungsgemäßen Stoffaustauschvorrichtung.

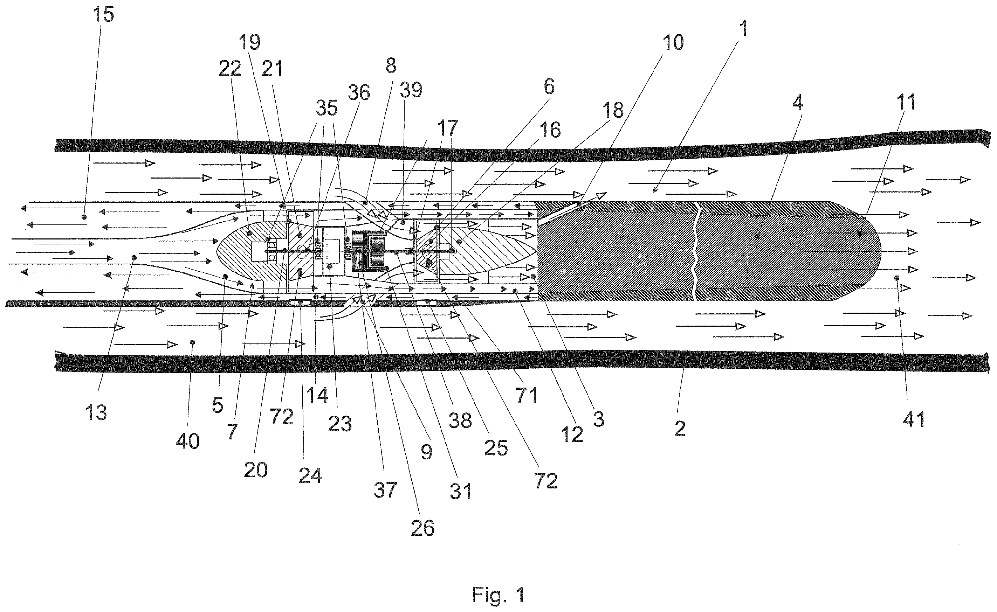

Fig. 1 zeigt eine Stoffaustauschvorrichtung 1 im Betrieb in einer intrakorporalen Anordnung. In dieser Anordnung wird die Stoffaustauschvorrichtung 1 als intravaskulärer Katheter in einem Blutgefäß 2 verwendet. Die Stoffaustauschvorrichtung 1 umfasst einen Hohlraum 3, eine Stoffaustauschmembran 4, eine Zuführleitung 5, eine Blutpumpe 6 und eine Antriebseinheit 7. Im Betrieb ist in dem Hohlraum 3 Blut aufgenommen. Der Hohlraum 3 weist zwei Bluteinlässe 8, 9 und zwei Blutauslässe 10, 11 auf. Die beiden Blutauslässe 10, 11 sind in unterschiedlichen Abständen von den beiden Bluteinlässen 8, 9 angeordnet. Der erste Blutauslass 10 ist unmittelbar nach der Blutpumpe 6 angeordnet und bildet einen Bypass für die Stoffaustauschmembran 4. Der zweite Blutauslass 11 ist nach der Stoffaustauschmembran 4 angeordnet und bildet das proximale Ende der Stoffaustauschvorrichtung 1. Die Stoffaustauschmembran 4 grenzt an den Hohlraum 3 an. Sie kann eine Hohlfasermembran mit zylinderförmig um den Hohlraum 3 angeordneten Fasern sein. Die Stoffaustauschmembran 4 behält im umgebenden Gefäß 2 im Wesentlichen eine konstante Stellung und wird diesem gegenüber nicht gedreht.

Die Zuführleitung 5 ist zur Zuführung eines Austauschfluids zu der Stoffaustauschmembran 4 eingerichtet. Das Austauschfluid kann beispielsweise ein CO2-armes Gasgemisch sein. Die Zuführleitung 5 verbindet beispielsweise den Einlass 12 einer Hohlfasermembran mit einem Zuführschlauch 13, der die Stoffaustauschvorrichtung 1 mit einer extrakorporalen Austauschvorrichtung zur Wiederaufbereitung des Austauschfluids verbindet. Die Stoffaustauschvorrichtung 1 umfasst außerdem eine Rückführleitung 14 zur Rückführung eines Austauschfluids von der Stoffaustauschmembran 4. Die Rückführleitung 14 sowie ein daran angeschlossener Rückführschlauch 15 sind eingerichtet, einem Unterdruck Stand zu halten. Der Zuführschlauch 13 und der Rückführschlauch 15 können als ein einziger mehrlumiger, z.B. doppelwandiger, Schlauch ausgebildet sein.

Die Blutpumpe 6 ist in dem Hohlraum 3 angeordnet. Sie ist zum Pumpen von Blut in Richtung von den Bluteinlässen 8, 9 zu den Blutauslässen 10, 11 des Hohlraums 3 eingerichtet. Die Blutpumpe 6 ist bevorzugt eine Kreiselpumpe in radialer, diagonaler oder axialer Ausführung. Ein Pumpenläufer 16 der Blutpumpe 6 ist in zumindest einem Gleitlager 17 gelagert. Es kann ein Leitrad 18 nach dem Pumpenläufer 16 zur Reduktion oder vollständigen Entfernung des rotatorischen Anteils des geförderten Mediums und Umwandlung dessen in eine Druckerhöhung angeordnet werden. Wird dieses Leitrad 18 jedoch nicht verwendet, kann auch die Strömung mit einer Rotation zu der Stoffaustauschmembran 4 geleitet werden, damit diese nicht (nur) in Längsrichtung angeströmt wird. Die Blutpumpe 6 hat die Aufgabe, den Blutfluss so zu fördern, dass die Stoffaustauschmembran 4 ausreichend mit Blut durchströmt wird. Im Fall einer intravaskulären Anwendung stellt die Stoffaustauschvorrichtung 1 einen zusätzlichen Widerstand im Gefäß 2 dar, welcher durch die von der Blutpumpe 6 dem Blut zugeführte Bewegungsenergie zumindest teilweise kompensiert wird. Gleichzeitig kann je nach Betriebsart der Druckverlust durch die Stoffaustauschvorrichtung 1 im Gefäß 2 ganz oder teilweise ausgeglichen oder auch eine Drucksteigerung im Gefäß 2 erreicht werden. Insbesondere kann dies durch den ersten Blutauslass 10 erfolgen, welcher ermöglicht, dass nicht jedes Blutvolumen, welches durch die Blutpumpe 6 strömt auch durch die Stoffaustauschmembran 4 strömt.

Die Antriebseinheit 7 dient dem Antrieb der Blutpumpe 6. Mit ihrer Hilfe kann die Blutpumpe in Rotation versetzt und so durch diese der Eintritt des Blutes in die Stoffaustauschmembran 4 definiert werden in Bezug auf Strömungsrichtung und Volumenstrom. Die Antriebseinheit 7 umfasst eine Turbine 19. Die Turbine 19 ist mit der Zuführleitung 5 verbunden, insbesondere in der Zuführleitung 5 angeordnet und mittels eines durch die Zuführleitung 5 zugeführten Austauschfluids antreibbar. Die Turbine 19 weist ein über eine drehbar gelagerte Welle 20 mit der Blutpumpe 6 gekoppeltes Laufrad 21 und ein vor dem Laufrad 21 angeordnetes Leitrad 22 auf. Die Blutpumpe 6 ist über ein Getriebe 23 mit der Turbine 19 gekoppelt. Das Getriebe 23 ist zur Herabsetzung der Drehzahl der Blutpumpe 6 gegenüber der Drehzahl der Turbine 19 eingerichtet. Es kann beispielsweise ein Planetengetriebe sein. Die Antriebseinheit 7 und die Blutpumpe 6 bilden zusammen eine Fördereinrichtung zur Förderung von Blut durch den Hohlraum 3 der Stoffaustauschvorrichtung 1. Im Betrieb wird der Pumpenläufer 16 der Blutpumpe 6 von der Turbine 19 derart angetrieben, dass eine Beschleunigung der Blutströmung im Bereich der Bluteinlässe 8, 9 und somit ein Überdruck am distalen Ende des Hohlraums 3 erzeugt wird. Die Drehzahl der Turbine 19 kann dabei über den Massenstrom bzw. den Volumenstrom des Austauschfluids so gesteuert werden, dass der erzielte Differenzdruck an der Pumpe alle auftretenden Druckverluste von den Bluteinlässen (8,9) bis zum proximalen Blutauslass (11) gerade ausgleicht. Dadurch wird effektiv der durch die Stoffaustauschmembran 4 verursachte Strömungswiderstand im Inneren der Stoffaustauschvorrichtung 1 kompensiert.

In der Stoffaustauschvorrichtung 1 sind zwei Drehzahlsensoren 24, 25 integriert. Der erste Drehzahlsensor 24 ist zur Aufnahme der Drehzahl der Turbine 19, der zweite Drehzahlsensor 25 zur Aufnahme der Drehzahl der Blutpumpe 6 eingerichtet. Beide Drehzahlsensoren 24, 25 sind Hallsensoren. Die Drehzahlsensoren 24, 25 sind in ein Gehäuse 71 der Stoffaustauschvorrichtung 1 integriert, welches die Antriebseinheit 7 und die Blutpumpe 6 umgibt. Als Signalgeber für die Drehzahlsensoren 24, 25 ist im Laufrad 21 der Turbine 19 und im Pumpenläufer 16 der Blutpumpe 6 jeweils ein Magnet 72, vorzugsweise zentrisch angeordnet (die exzentrische Darstellung ist nur schematisch und dient der einfacheren Erkennbarkeit; in der Praxis ist eine Unwucht zu vermeiden) .

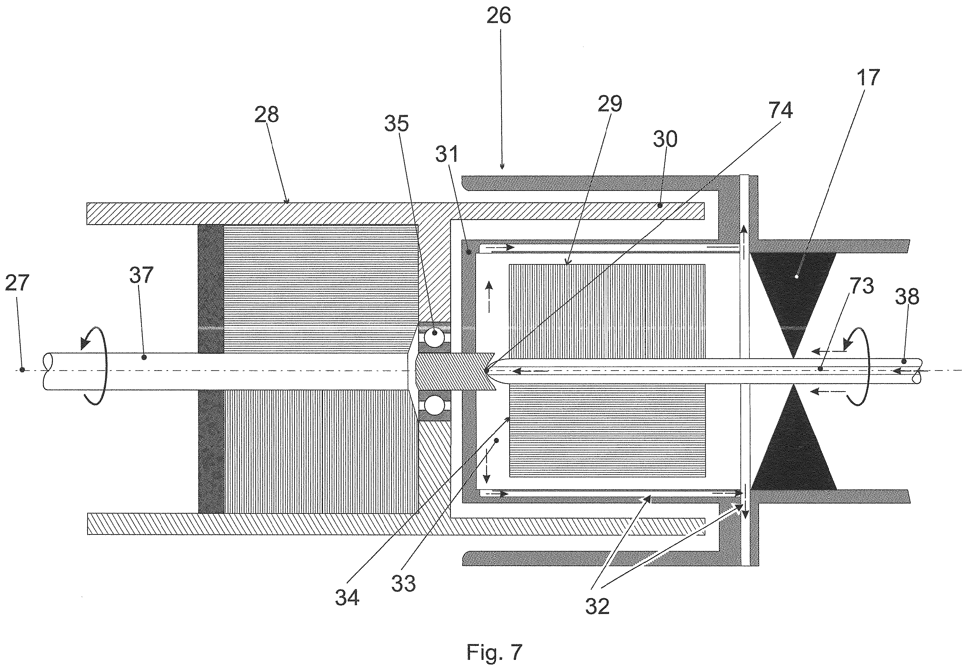

Ein Pumpenläufer 16 der Blutpumpe 6 ist über eine Magnetkupplung 26 mit der Turbine 19 verbunden (vgl. Fig. 7). Die Magnetkupplung 26 weist zur Drehmomentübertragung entlang einer Drehachse 27 zwei relativ zueinander drehbare Kupplungsteile 28, 29 mit jeweils einem zweipoligen Dauermagnet auf. Einer der Kupplungsteile 28 umfasst ein zumindest teilweise ferromagnetisches, topfförmiges Umleitelement 30. Das Umleitelement 30 ist mit dem Dauermagnet des antriebsseitigen Kupplungsteils 28 drehfest verbunden. Ein Teil des Umleitelements 30 ist radial außerhalb des Dauermagnets des anderen Kupplungsteils 29 angeordnet. Der Mantel des Umleitelements 30 ist lediglich in einem schmalen Winkelbereich durch eine diamagnetische oder paramagnetische Trennung (nicht dargestellt) unterbrochen. Die diamagnetische Trennung teilt das Umleitelement 30 im Wesentlichen in zwei ferromagnetische Hälften bzw. Halbschalen. Eine durch die diamagnetische oder parmagnetische Trennung verlaufende Schnittebene steht somit senkrecht auf eine Magnetisierungsrichtung des mit dem Umleitelement 30 verbundenen antriebsseitigen zweipoligen Dauermagnets. Die durch die diamagnetische oder paramagnetische Trennung definierten ferromagnetischen Abschnitte des Umleitelements 30 sind daher entsprechend dem antriebsseitigen Dauermagnet magnetisiert.

Aufgrund der berührungslosen Kopplung ist zwischen dem antriebsseitigen Kupplungsteil 28 und dem abtriebsseitigen Kupplungsteil 29 eine hermetische Trennwand 31 vorgesehen. Innerhalb der hermetischen Trennwand 31 ist zumindest ein Spülkanal 32 vorgesehen. Die Spülkanäle 32 verbinden einen Spalt 33 zwischen der Stirnseite 34 des pumpenseitigen Kupplungsteils 29 und der hermetischen Trennwand 31 mit einem den Bluteinlässen 8, 9 angrenzenden Bereich. Ein zusätzlicher Spülkanal 73 ist innerhalb des pumpenseitigen Wellenabschnitts 38 zur Schmierung einer feststehenden Gleitlagerpfanne 74, in der der Wellenabschnitt 38 gelagert ist, vorgesehen. Wie in Fig. 7 im Detail ersichtlich, sind die drehbaren Komponenten der Stoffaustauschvorrichtung auf der Abtriebsseite (auch Pumpenseite oder "Blutseite") in Gleitlagern 17 gelagert und die drehbaren Komponenten der Stoffaustauschvorrichtung auf der Antriebsseite (auch Turbinenseite) sind in Wälzlagern 35 gelagert (vgl. auch Fig. 1).

Im Betrieb wird durch die Strömung des zugeführten Austauschfluids über die Schaufeln der Turbine 19 ein Drehmoment auf das Laufrad 21 aufgebracht. Die Turbine 19 überträgt das Drehmoment über die drehbar in Wälzlagern 35 gelagerte Welle 36 auf die Antriebsseite des Getriebes 23. An der Abtriebsseite des Getriebes 23 wird ein entsprechend höheres Drehmoment bei geringerer Drehzahl über einen weiteren Wellenabschnitt 37 auf den antriebsseitigen Kupplungsteil 28 der Magnetkupplung 26 übertragen. Ist das Antriebsfluid ein flüssiges Medium kann auf das Getriebe 23 verzichtet werden, vorausgesetzt die Turbine und die Pumpe haben beim jeweiligen ausgelegten Betriebspunkt dieselbe Drehzahl und das abgegeben Drehmoment der Turbine entspricht dem benötigten Eingangsdrehmoment der Pumpe. Durch die magnetischen Kräfte zwischen den Kupplungsteilen 28, 29 wird das Drehmoment von dem antriebsseitigen Kupplungsteil 28 auf den abtriebsseitigen Kupplungsteil 29 übertragen, wobei die Stärke der magnetischen Kräfte ein bestimmtes maximal übertragbares Drehmoment definiert, jenseits dessen es zu einem "durchdrehen" der Kupplungsteile 28, 29 relativ zueinander kommt. Das abtriebsseitige Kupplungsteil 29 überträgt ein von dem antriebsseitigen Kupplungsteil 28 ausgeübtes Drehmoment über einen dritten, in Gleitlagern 17 gelagerten Wellenabschnitt 38 auf den Pumpenläufer 16 der Blutpumpe 6. Der Pumpenläufer 16 fördert das durch ein (optionales) Vorleitrad 39 anströmende Blut 40 von den Bluteinlässen 8, 9 durch den Hohlraum 3 in Richtung der Stoffaustauschmembran 4 der Stoffaustauschvorrichtung 1. Die Fördereinrichtung erzeugt auf diese Weise eine Druckdifferenz zwischen den Bluteinlässen 8, 9 und dem proximalen Ende des Hohlraums 3, welche vorzugsweise einen Druckverlust zwischen dem proximalen und dem distalen Ende der Stoffaustauschvorrichtung 1 aufgrund des Strömungswiderstands der Stoffaustauschmembran 4 im Wesentlichen vollständig kompensiert, sodass das nach der Stoffaustauschvorrichtung 1 im Gefäß 2 strömende Blut 41 zumindest dieselbe innere Energie aufweist wie das Blut 40 davor. Die Konzentration eines Stoffes kann sich an der Stelle im Blut 40 zu der Stelle im Blut 41 entweder reduziert (z.B. CO₂ Reduktion) oder erhöht (z.B. O₂ Anreicherung) haben.

Anstelle einer Hohlfasermembran kann auch ein anderer Membrantyp in der Stoffaustauschvorrichtung 1 als Stoffaustauschmembran 4 zum Einsatz kommen, wobei der Fachmann die Fördereinrichtung mit der Antriebseinheit 7 und der Blutpumpe 6 an die zu erwartende Druckdifferenz aufgrund des unterschiedlichen Strömungswiderstands anderer Membrantypen anpassen wird.

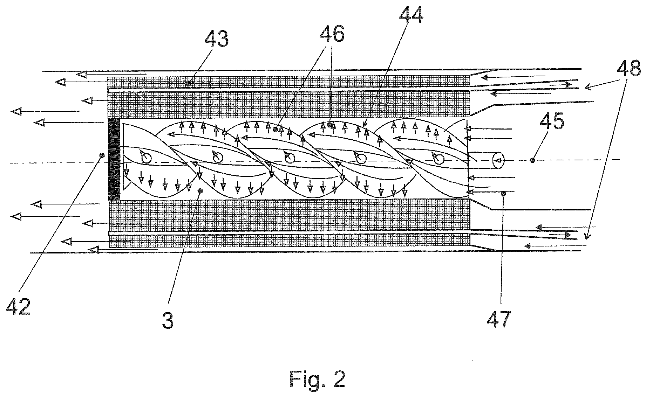

Bei dem in Fig. 2 gezeigten Abschnitt eines Ausführungsbeispiels ist zwischen der Blutpumpe (auf der rechten Seite, hier nicht dargestellt) und einem Blutauslass 42 im Hohlraum 3 innerhalb einer Stoffaustauschmembran 43 eine Umlenkeinrichtung 44 angeordnet. Die Umlenkeinrichtung 44 ist zur teilweisen Umlenkung eines axial durch den Hohlraum 3 strömenden Blutstroms in radiale Richtung eingerichtet. Die in Fig. 2 schematisch dargestellte Umlenkeinrichtung 44 weist konzentrisch zu einer Längsachse 45 zwischen der Blutpumpe und dem Blutauslass 42 spiralförmige Leitflächen 46 auf. Die Umlenkeinrichtung 44 kann starr im Hohlraum 3 der Stoffaustauschvorrichtung 1 befestigt oder aber im Hohlraum 3 drehbar gelagert sein. Durch die spiralförmige Leitflächen 46 wird das im Betrieb von der Pumpe zuströmende Blut 47 im Zentrum entlang der Längsachse 45 durch die Spirale immerfort radial über die ausgeführte Länge und durch die spiralförmige Form somit quer zur Stoffaustauschmembran 43 gedrängt. In der Stoffaustauschmembran 43 findet ein Stoffaustausch mit einem Austauschfluid statt, welches durch Zu- und Rückführleitungen 48 der Stoffaustauschmembran 43 zugeführt bzw. von der Stoffaustauschmembran 43 rückgeführt wird.

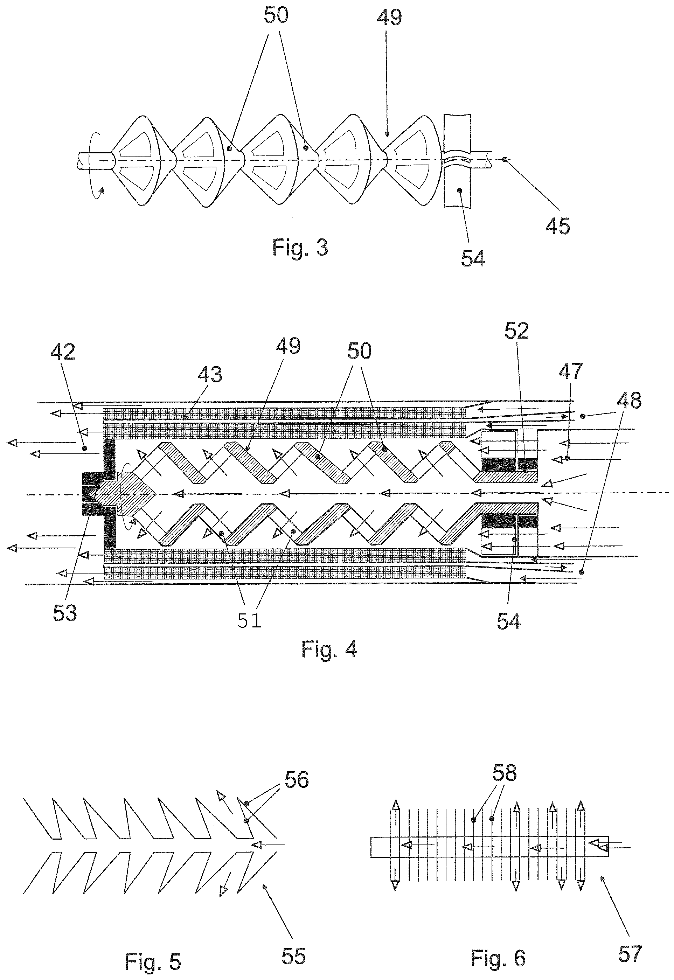

In Fig. 3 und 4 ist eine weitere Variante einer Umlenkeinrichtung 49 schematisch dargestellt. Die Stoffaustauschmembran 43 sowie die Zu- und Rückführleitungen 48 entsprechen jenen aus Fig. 2. Die Umlenkeinrichtung 49 gemäß Fig. 3 und 4 weist konzentrisch zu einer Längsachse 45 zwischen der Blutpumpe (nicht gezeigt; rechte Seite in Fig. 4) und dem Blutauslass 42 kegelstumpfförmige Leitflächen 50 auf. Das im Betrieb von der Pumpe zuströmende Blut 47 strömt entlang der Außenseite der Leitflächen 50 durch die dazwischen frei gelassenen Abstände bzw. Öffnungen 51 der Umlenkeinrichtung 49 und durch die Stoffaustauschmembran 43. Die Umlenkeinrichtung 49 ist frei drehbar zwischen zwei Gleitlagern 52, 53 gelagert. Das stromabwärts angeordnete Gleitlager 53 weist Spülkanäle auf, um im Lagerbereich eine Schmierung zu gewährleisten. Die Umlenkeinrichtung 49 weist außerdem ein eigenes Turbinenelement 54 auf, welches durch das von der Blutpumpe heranströmenden Blut 47 angetrieben wird und so die Umlenkeinrichtung 49 in Drehung versetzt. Mittels der durch die Drehung erzeugten Zentrifugalkraft wird eine zusätzliche Beschleunigung des Blutstroms in radiale Richtung durch die radial außerhalb angeordnete Stoffaustauschmembran 43 erzielt. Ein Teil des Bluts strömt im Zentrum der innen durchgängig hohlen Umlenkeinrichtung 49 entlang der Längsachse 45.

Fig. 5 zeigt stark vereinfacht eine ähnlich funktionierende Umlenkeinrichtung 55 mit pfeilförmigen Leitflächen und Fig. 6 ebenfalls stark vereinfacht eine Umlenkeinrichtung 56 mit scheibenförmigen Leitflächen. Die Umlenkeinrichtungen 44, 49, 55, 57 können jeweils frei drehbar oder optional mit dem Pumpenläufer 16 der Blutpumpe 6 drehfest gekoppelt sein und sich mit dessen Drehzahl mitdrehen, um mittels einer durch die Drehung erzeugten Zentrifugalkraft eine Beschleunigung des Blutstroms in radiale Richtung durch eine radial außerhalb angeordnete Stoffaustauschmembran 43 zu erzielen. Alternativ zur Kopplung mit dem Pumpenläufer 16 kann jeweils ein Turbinenelement 54 (vgl. Fig. 4) in die Umlenkeinrichtung integriert sein.

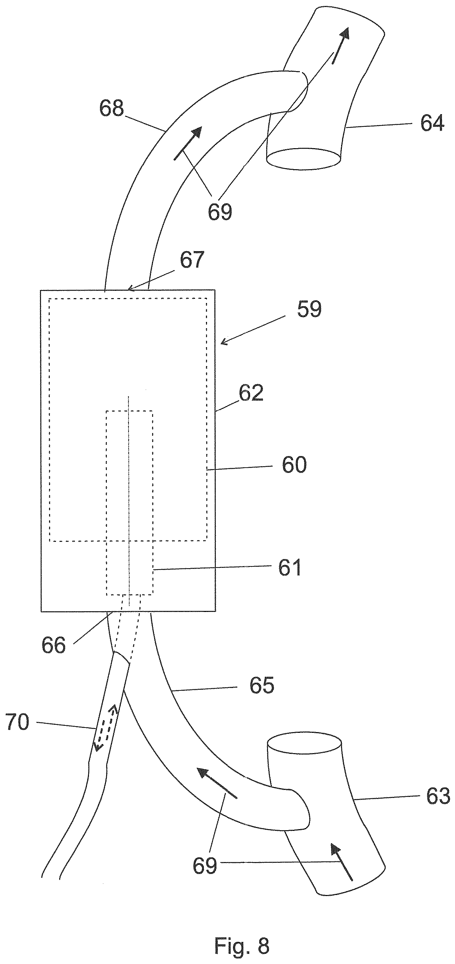

In der in Fig. 8 gezeigten Verwendung ist eine erfindungsgemäße Stoffaustauschvorrichtung 59 (nur schematisch angedeutet) mit einer Stoffaustauschmembran 60 und einer Fördereinrichtung 61 in einem körperfremden Schlauch 62 angeordnet, welcher zwei Blutgefäße 63, 64 miteinander verbindet. Durch einen ersten Schlauchabschnitt 65 wird ein Teil des Blutstroms oder der gesamte Blutstrom aus dem ersten Blutgefäß 63 entnommen und durch einen Bluteinlass 66 der Stoffaustauschvorrichtung 59 der Fördereinrichtung 61 mit einer Turbine, einer Magnetkupplung und einer Blutpumpe und der Stoffaustauschmembran 60 der Stoffaustauschvorrichtung 59 zugeführt. Vom Blutauslass 67 der Stoffaustauschvorrichtung 59 wird das Blut durch einen zweiten Schlauchabschnitt 68 dem zweiten Blutgefäß 64 zugeführt. Das zweite Blutgefäß 64 kann mit dem ersten Blutgefäß 63 identisch sein. Die Strömungsrichtung des Bluts ist durch Pfeile 69 angedeutet. Durch einen Multilumenschlauch 70 wird der Stoffaustauschvorrichtung 59 ein Austauschfluid zugeführt und von der Stoffaustauschvorrichtung 59 rückgeführt. Bei dieser Anwendung kann auf ein Getriebe zwischen der Turbine und der Blutpumpe verzichtet werden, da bei Verwendung eines Gases als Antriebsfluid für die Turbine auf Grund der platzverhältnisse dementsprechend größer gestaltet werden kann. Vorausgesetzt, die Turbine und die Pumpe haben beim jeweiligen ausgelegten Betriebspunkt dieselbe Drehzahl und das abgegeben Drehmoment der Turbine entspricht dem benötigten Eingangsdrehmoment der Pumpe.

## Patentansprüche

1. Stoffaustauschvorrichtung (1) zur intravenösen Verwendung umfassend einen Hohlraum (3) zur Aufnahme von Blut mit zumindest einem Bluteinlass (8, 9) und zumindest einem Blutauslass (10, 11), eine an den Hohlraum (3) angrenzende Stoffaustauschmembran (4), eine Zuführleitung (5) zur Zuführung eines Austauschfluids zu der Stoffaustauschmembran (4), eine in dem Hohlraum (3) angeordnete Blutpumpe (6) und eine Antriebseinheit (7) für die Blutpumpe (6), wobei die Blutpumpe (6) zum Pumpen von Blut in Richtung von einem Bluteinlass (8, 9) zu einem Blutauslass (10, 11) des Hohlraums (3) eingerichtet ist, **dadurch gekennzeichnet, dass** die Antriebseinheit (7) eine Turbine (19) umfasst, welche mit der Zuführleitung (5) verbunden und mittels eines durch die Zuführleitung (5) zugeführten Austauschfluids antreibbar ist, wobei die Turbine (19) mindestens ein mit der Blutpumpe (6) gekoppeltes Laufrad (21) und ein vor dem Laufrad (21) angeordnetes Leitrad (22) aufweist.

2. Stoffaustauschvorrichtung (1) nach Anspruch 1, **dadurch gekennzeichnet, dass** ein Pumpenläufer (16) der Blutpumpe (6) in einem Gleitlager (17) gelagert ist.

3. Stoffaustauschvorrichtung (1) nach Anspruch 1 oder 2, **dadurch gekennzeichnet, dass** in der Stoffaustauschvorrichtung (1) zumindest ein Drehzahlsensor (24, 25) integriert ist, welcher zur Aufnahme der Drehzahl der Turbine (19), der Blutpumpe (6) oder einer Kupplung zwischen Blutpumpe (6) und Turbine (19) eingerichtet ist.

4. Stoffaustauschvorrichtung (1) nach einem der Ansprüche 1 bis 3, **dadurch gekennzeichnet, dass** die Blutpumpe (6) über ein Getriebe (23) mit der Turbine (19) gekoppelt ist, wobei das Getriebe (23) zur Herabsetzung der Drehzahl der Blutpumpe (6) gegenüber der Drehzahl der Turbine (19) eingerichtet ist.

5. Stoffaustauschvorrichtung (1) nach einem der Ansprüche 1 bis 4, **gekennzeichnet durch** eine Rückführleitung (15) zur Rückführung eines Austauschfluids von der Stoffaustauschmembran (4) und/oder der Turbine (19), wobei die Rückführleitung (15) eingerichtet ist, einem Unterdruck stand zu halten.

6. Stoffaustauschvorrichtung (1) nach einem der Ansprüche 1 bis 5, **dadurch gekennzeichnet, dass** ein Pumpenläufer (16) der Blutpumpe (6) über eine Magnetkupplung (26) mit der Turbine (19) verbunden ist, wobei die Magnetkupplung (26) zur Drehmomentübertragung entlang einer Drehachse zwei relativ zueinander drehbare Kupplungsteile (28, 29) mit jeweils einem Dauermagnet aufweist.

7. Stoffaustauschvorrichtung (1) nach Anspruch 6, **dadurch gekennzeichnet, dass** einer der Kupplungsteile (28) ein zumindest teilweise ferromagnetisches Umleitelement (30) umfasst, welches mit dem Dauermagnet des Kupplungsteils (28) drehfest verbunden ist, wobei ein Teil des Umleitelements (30) radial außerhalb des Dauermagnets des anderen Kupplungsteils (29) angeordnet ist.

8. Stoffaustauschvorrichtung (1) nach Anspruch 7, **dadurch gekennzeichnet, dass** das zumindest teilweise ferromagnetische Umleitelement (30), zumindest eine diamagnetische oder paramagnetische Trennung aufweist.

9. Stoffaustauschvorrichtung (1) nach einem der Ansprüche 6 bis 8, **dadurch gekennzeichnet, dass** zwischen den beiden Kupplungsteilen (28, 29) eine hermetische Trennwand (31) angeordnet ist, welche die Antriebseinheit (7) und die Blutpumpe (6) hermetisch voneinander trennt.

10. Stoffaustauschvorrichtung (1) nach Anspruch 9, **dadurch gekennzeichnet, dass** eine integrierte Lagerung, vorzugsweise ein Wälzlager (35), zwischen einem antriebsseitigen Kupplungsteil (28) und der hermetische Trennwand (31) und eine Gleitlagerung (74) zwischen der hermetischen Trennwand (31) und dem abtriebsseitigen Kupplungsteil (29) vorgesehen sind.

11. Stoffaustauschvorrichtung (1) nach einem der Ansprüche 7 bis 10, **dadurch gekennzeichnet, dass** das Umleitelement (30) mit dem antriebsseitigen Kupplungsteil (28) drehfest verbunden ist, wobei innerhalb einer hermetischen Trennwand (31) zwischen den beiden Kupplungsteilen (28, 29) zumindest ein Spülkanal (32) vorgesehen ist, welcher einen Spalt (33) zwischen der Stirnseite (34) des pumpenseitigen Kupplungsteils (29) und der hermetischen Trennwand (31) im Betrieb mit zumindest einem Blutstrom außerhalb der hermetischen Trennwand (31) verbindet, wobei dieser Blutstrom entweder ein Blutstrom durch den Bluteinlass (8, 9) oder ein Blutstrom (40) vor dem Bluteinlass (8, 9) sein kann.

12. Stoffaustauschvorrichtung (1) nach einem der Ansprüche 1 bis 11, **dadurch gekennzeichnet, dass** zwischen der Blutpumpe (6) und dem zumindest einen Blutauslass (11) im Hohlraum (3) eine Umlenkeinrichtung (44, 49, 55, 57) angeordnet ist, wobei die Umlenkeinrichtung (44, 49, 55, 57) zur teilweisen Umlenkung eines axial durch den Hohlraum (3) strömenden Blutstroms in radiale Richtung und / oder zur Induzierung von Turbulenz in eben diesem Blutstrom eingerichtet ist.

13. Stoffaustauschvorrichtung (1) nach Anspruch 12, **dadurch gekennzeichnet, dass** die Umlenkeinrichtung (44, 49, 55, 57) konzentrisch zu einer Längsachse (45) zwischen der Blutpumpe (6) und dem Blutauslass (11) spiralförmige, kegelförmige, pfeilförmige und/oder scheibenförmige Leitflächen (46, 50, 56, 58) aufweist.

14. Stoffaustauschvorrichtung (1) nach Anspruch 12 oder 13, **dadurch gekennzeichnet, dass** die Umlenkeinrichtung (44, 49, 55, 57) im Hohlraum (3) drehbar gelagert ist.

15. Stoffaustauschvorrichtung (1) nach einem der Ansprüche 1 bis 14, **dadurch gekennzeichnet, dass** der Hohlraum (3) zumindest zwei Blutauslässe (10, 11) in unterschiedlichen Abständen von dem zumindest einen Bluteinlass (8, 9) aufweist.

## Claims

1. A substance exchange device (1) for intravenous use, comprising a cavity (3) for receiving blood having at least one blood inlet (8, 9) and at least one blood outlet (10, 11), a substance exchange membrane (4) adjoining the cavity (3), a supply duct (5) for supplying an exchange fluid to the substance exchange membrane (4), a blood pump (6) arranged within the cavity (3) and a drive unit (7) for the blood pump (6), wherein the blood pump (6) is configured to pump blood in a direction from a blood inlet (8, 9) to a blood outlet (10, 11) of the cavity (3), **characterised in that** the drive unit (7) comprises a turbine (19), which is connected to the supply duct (5) and may be driven by an exchange fluid supplied via the supply duct (5), wherein the turbine (19) comprises at least a rotor (21) coupled to the blood pump (6) and an idler (22) arranged upstream of the rotor (21).

2. The substance exchange device (1) according to claim 1, **characterised in that** a pump rotor (16) of the blood pump (6) is supported in a sliding contact bearing (17).

3. The substance exchange device (1) according to claim 1 or 2, **characterised in that** at least one speed sensor (24, 25) is integrated with the substance exchange device (1), which speed sensor (24, 25) is configured to sense the speed of the turbine (19), the blood pump (6) or a coupling between the blood pump (6) and the turbine (19).

4. The substance exchange device (1) according to any one of claims 1 to 3, **characterised in that** the blood pump (6) is coupled to the turbine (19) via a gearing (23), wherein the gearing (23) is configured to reduce the speed of the blood pump (6) with respect to the speed of the turbine (19).

5. The substance exchange device (1) according to any one of claims 1 to 4, **characterised by** a return duct (15) for returning an exchange fluid from the substance exchange membrane (4) and/ or the turbine (19), wherein the return duct (15) is configured to withstand a negative pressure.

6. The substance exchange device (1) according to any one of claims 1 to 5, **characterised in that** a pump rotor (16) of the blood pump (6) is connected to the turbine (19) via a magnetic coupling (26), wherein the magnetic coupling (26) comprises two coupling parts (28, 29) for torque transmission along an axis of rotation, said coupling parts (28, 29) being rotatable relative to each other and each having a permanent magnet.

7. The substance exchange device (1) according to claim 6, **characterised in that** one of the coupling parts (28) comprises an at least partially ferromagnetic guiding element (30) which is non-rotatably connected to the permanent magnet of the coupling part (28), wherein one part of the guiding element (30) is disposed radially outside of the permanent magnet of the other coupling part (29).

8. The substance exchange device (1) according to claim 7, **characterised in that** the at least partially ferromagnetic guiding element (30) comprises at least one diamagnetic or paramagnetic separation.

9. The substance exchange device (1) according to any one of claims 6 to 8, **characterised in that** between the two coupling parts (28, 29) a hermetic separating wall (31) is arranged, which separates the drive unit (7) and the blood pump (6) hermetically from one another.

10. The substance exchange device (1) according to claim 9, **characterised in that** an integrated bearing, preferably a rolling contact bearing (35), between a drive-side coupling part (28) and the hermetic separating wall (31), and a sliding contact bearing (74) between the hermetic separating wall (31) and the output-side coupling part (29) are provided.

11. The substance exchange device (1) according to any one of claims 7 to 10, **characterised in that** the guiding element (30) is non-rotatably connected to the drive-side coupling part (28), wherein within a hermetic separating wall (31) between the two coupling parts (28, 29) at least one flushing duct (32) is provided, which connects, during operation, a gap (33) between the front side (34) of the pump-side coupling part (29) and the hermetic separating wall (31) to at least one blood flow outside of the hermetic separating wall (31), wherein said blood flow may be either a blood flow through the blood inlet (8, 9) or a blood flow (40) upstream of the blood inlet (8, 9).

12. The substance exchange device (1) according to any one of claims 1 to 11, **characterised in that** between the blood pump (6) and the at least one blood outlet (11) in the cavity (3) a diverting member (44, 49, 55, 57) is arranged, wherein the diverting member (44, 49, 55, 57) is configured to partially divert in the radial direction a blood flow flowing axially through the cavity (3) and/or to induce turbulences in this very blood flow.

13. The substance exchange device (1) according to claim 12, **characterised in that** the diverting member (44, 49, 55, 57) comprises helical, conical, arrow-shaped and/or disc-shaped guiding surfaces (46, 50, 56, 58), which are concentric with respect to a longitudinal axis (45) between the blood pump (6) and the blood outlet (11).

14. The substance exchange device (1) according to claim 12 or 13, **characterised in that** the diverting member (44, 49, 55, 57) is supported rotatably within the cavity (3).

15. The substance exchange device (1) according to any one of claims 1 to 14, **characterised in that** the cavity (3) comprises at least two blood outlets (10, 11) at different distances from the at least one blood inlet (8, 9).

## Revendications

1. Dispositif d'échange de substances (1) pour utilisation intraveineuse comprenant une cavité (3) pour recevoir du sang avec au moins une entrée de sang (8, 9) et au moins une sortie de sang (10, 11), une membrane d'échange de substances (4) adjacente à la cavité (3), une conduite d'alimentation (5) pour alimenter un fluide d'échange à la membrane d'échange de substances (4), une pompe à sang (6) disposée dans la cavité (3) et une unité d'entraînement (7) pour la pompe à sang (6), la pompe à sang (6) étant conçue pour pomper le sang dans le sens d'une entrée de sang (8, 9) vers une sortie de sang (10, 11) de la cavité (3), **caractérisé en ce que** l'unité d'entraînement (7) comprend une turbine (19) connectée à la conduite d'alimentation (5) et pouvant être entraînée par un fluide d'échange circulant dans la conduite d'alimentation (5), la turbine (19) comprenant au moins une roue à aubes (21) couplée à la pompe à sang (6) et une roue directrice (22) disposée en amont de la roue à aubes (21).

2. Dispositif d'échange de substances (1) selon la revendication 1, **caractérisé en ce qu'**un rotor de pompe (16) de la pompe à sang (6) est monté dans un palier lisse (17).

3. Dispositif d'échange de substances (1) selon la revendication 1 ou 2, **caractérisé en ce qu'**au moins un capteur de vitesse (24, 25) est intégré dans le dispositif d'échange de substances (1), lequel est conçu pour détecter la vitesse de rotation de la turbine (19), de la pompe à sang (6) ou d'un couplage entre la pompe à sang (6) et la turbine (19).

4. Dispositif d'échange de substances (1) selon l'une quelconque des revendications 1 à 3, **caractérisé en ce que** la pompe à sang (6) est couplée à la turbine (19) par un engrenage (23), l'engrenage (23) étant conçu pour réduire la vitesse de rotation de la pompe à sang (6) par rapport à la vitesse de rotation de la turbine (19).

5. Dispositif d'échange de substances (1) selon l'une quelconque des revendications 1 à 4, **caractérisé par** une conduite de retour (15) pour renvoyer un fluide d'échange de la membrane d'échange de substances (4) et/ou de la turbine (19), la conduite de retour (15) étant conçue pour résister à une dépression.

6. Dispositif d'échange de substances (1) selon l'une quelconque des revendications 1 à 5, **caractérisé en ce qu'**un rotor de pompe (16) de la pompe à sang (6) est relié à la turbine (19) par un couplage magnétique (26), le couplage magnétique (26) comprenant deux parties de couplage (28, 29) rotatives l'une par rapport à l'autre, chacune comprenant un aimant permanent.

7. Dispositif d'échange de substances (1) selon la revendication 6, **caractérisé en ce qu'**une des parties de couplage (28) comprend un élément de dérivation (30) au moins partiellement ferromagnétique, lequel est fixé de manière rotative avec l'aimant permanent de la partie de couplage (28), une partie de l'élément de dérivation (30) étant disposée radialement à l'extérieur de l'aimant permanent de l'autre partie de couplage (29).

8. Dispositif d'échange de substances (1) selon la revendication 7, **caractérisé en ce que** l'élément de dérivation ferromagnétique (30) comprend au moins une séparation diamagnétique ou paramagnétique.

9. Dispositif d'échange de substances (1) selon l'une quelconque des revendications 6 à 8, **caractérisé en ce qu'**une paroi d'étanchéité hermétique (31) est disposée entre les deux parties de couplage (28, 29), laquelle sépare hermétiquement l'unité d'entraînement (7) et la pompe à sang (6).

10. Dispositif d'échange de substances (1) selon la revendication 9, **caractérisé en ce qu'**un roulement intégré, de préférence un roulement à billes (35), est prévu entre une partie de couplage côté entraînement (28) et la paroi d'étanchéité hermétique (31), et un palier lisse (74) entre la paroi d'étanchéité hermétique (31) et la partie de couplage côté entraîné (29).

11. Dispositif d'échange de substances (1) selon l'une quelconque des revendications 7 à 10, **caractérisé en ce que** l'élément de dérivation (30) est fixé de manière rotative avec la partie de couplage côté entraînement (28), au moins un canal de rinçage (32) étant prévu à l'intérieur de la paroi d'étanchéité hermétique (31) entre les deux parties de couplage (28, 29), lequel relie un espace (33) entre la face d'extrémité (34) de la partie de couplage côté pompe (29) et la paroi d'étanchéité hermétique (31) à un flux sanguin à l'extérieur de la paroi d'étanchéité hermétique (31) en fonctionnement, ce flux sanguin étant soit un flux sanguin à travers l'entrée de sang (8, 9), soit un flux sanguin (40) en amont de l'entrée de sang (8, 9).

12. Dispositif d'échange de substances (1) selon l'une quelconque des revendications 1 à 11, **caractérisé en ce qu'**un dispositif de déviation (44, 49, 55, 57) est disposé entre la pompe à sang (6) et au moins une sortie de sang (11) dans la cavité (3), le dispositif de déviation (44, 49, 55, 57) étant conçu pour dévier partiellement un flux sanguin axialement circulant dans la cavité (3) en direction radiale et/ou pour induire une turbulence dans ce flux sanguin.

13. Dispositif d'échange de substances (1) selon la revendication 12, **caractérisé en ce que** le dispositif de déviation (44, 49, 55, 57) comprend des surfaces de guidage spiralées, coniques, en forme de flèche et/ou en forme de disque (46, 50, 56, 58) concentriques à un axe longitudinal (45) entre la pompe à sang (6) et la sortie de sang (11).

14. Dispositif d'échange de substances (1) selon la revendication 12 ou 13, **caractérisé en ce que** le dispositif de déviation (44, 49, 55, 57) est monté de manière rotative dans la cavité (3).

15. Dispositif d'échange de substances (1) selon l'une quelconque des revendications 1 à 14, **caractérisé en ce que** la cavité (3) comprend au moins deux sorties de sang (10, 11) à des distances différentes d'au moins une entrée de sang (8, 9).
